# EUROPEAN PATENT SPECIFICATION

(11) **EP 1 460 070 B1**
(45) Date of publication and mention of the grant of the patent: **16.05.2007**
(21) Application number: 03006253.3
(22) Date of filing: 20.03.2003
(51) Int. Cl.: A61K 31/451, A61K 31/4709, A61K 31/4725, A61K 31/497, A61P 3/04, A61P 15/00, A61P 25/22, A61P 25/24

(54) **Substituted piperidine and piperazine derivatives as melanocortin-4 receptor modulators**
Substituierte Piperidin- und Piperazin-Derivative als Melanocortin-4 Receptor Modulatoren
Dérivés substitués de la pipéridine et la pipérazine comme modulateurs du récepteur de la melanocortine-4

(43) Date of publication of application: 22.09.2004
(73) Proprietor: Santhera Pharmaceuticals (Schweiz) AG, 4410 Liestal (CH)
(72) Inventor: Soeberdt, Michael, 79618 Rheinfelden (DE); Weyermann, Philipp, 4450 Sissach (CH); Sprecher von, Andreas, 4104 Oberwill (CH)
(74) Representative: Grünecker, Kinkeldey, Stockmair & Schwanhäusser Anwaltssozietät

(56) References cited:
- WO-A-02/070511
- WO-A-03/009847

## Description

### Field of Invention

The present invention relates to *the use of* substituted piperidine and piperazine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation, and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified, and these are expressed in different tissues.

MC-1R was first found in melanocytes. Naturally occurring inactive variants of MC-1R in animals were shown to lead to alterations in pigmentation and a subsequent lighter coat color by controlling the conversion of phaeomelanin to eumelanin through the control of tyrosinase. From these, and other studies, it is evident that MC-1 R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain. (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration, and a dramatic reduction in elevated levels of cytokines and other mediators to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be in part mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands", Brain Research, 80: 302-306 (1998)). Further evidence for the involvement of MC-Rs in obesity includes: a) the agouti (A^{vy}) mouse which ectopically expresses an antagonist of the MC-1 R, MC-3R and MC-4R is obese, indicating that blocking the action of these three MC-Rs can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R and -5R agonist) injected intracerebroventricularly (ICV) in rodents, reduces food intake in several animal feeding models (NPY, ob/ob, agouti, fasted), while ICV injected SHU-9119 (MC-3R and -4R antagonist; MC-1R, and -5R agonist) reverses this effect and can induce hyperphagia; 4) chronic intraperitoneal treatment of Zucker fatty rats with an α-NDP-MSH derivative (HP-228) has been reported to activate MC1-R, -3R, -4R and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, 1998). Activation of melanocortin receptors of the brain appears to cause normal stimulation of sexual arousal. Evidence for the involvement of MC-R in male and/or female sexual dysfunction is detailed in WO/0074679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes, which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF), (Owen MJ and Nemeroff CB (1991). Physiology and pharmacology of corticotrophin releasing factor. *Pharmacol Rev* 43: 425 - 473). In addition to activation of the brain CRF system, there are several lines of evidence that melanocortins (MCs), which stem from propiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(*S*)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003)304(2), 818-26).

Chronic diseases such as malignant tumors or infections are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. *et al*. Role of the central melanocortin system in cachexia. Cancer Res. (2001) 61: 1432-1438).

*WO 03*/*009847 A1 describes phenylpiperidinyl-phenylalanine derivatives for the treatment of obesity.*

*WO 02*/*070511 A1 describes phenylpiperazinyl-phenylalanine amides, phenylpiperidinyl-phenylalanine amides and cyclohexyl-phenylalanine amides as modulators of melanocortin receptors 1 and 4.*

In view of the unresolved deficiencies in treatment of various diseases and disorders as discussed above, it is an object of the present invention to provide novel substituted piperidine and piperazine derivatives with improved ability to cross the blood brain barrier, which are useful as melanocortin-4 receptor modulators to treat cancer cachexia, muscle wasting, anorexia, anxiety, depression, obesity, diabetes, sexual dysfunction, and other diseases with MC-4R involvement.

### Summary of the Invention

The present invention relates to substituted piperidine and piperazine derivatives of the following general structural formula *(III) for use as a medicament*.

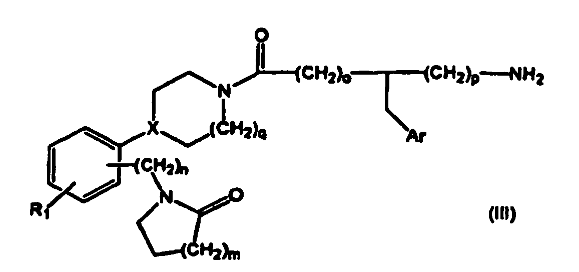

*The compounds according to formula (III) are intermediates in the preparation of compounds according to formula (I) and formula (II) which are shown below. Compounds according to formula (I) and formula (II) are not part of the invention.*

These piperidine and piperazine derivatives are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes, and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to substituted piperidine and piperazine derivatives according to formula (III) which are useful as melanocortin receptor modulators, in particular, selective MC-4R agonists and MC-4R antagonists.

The compounds are represented by the following structural formula (III) 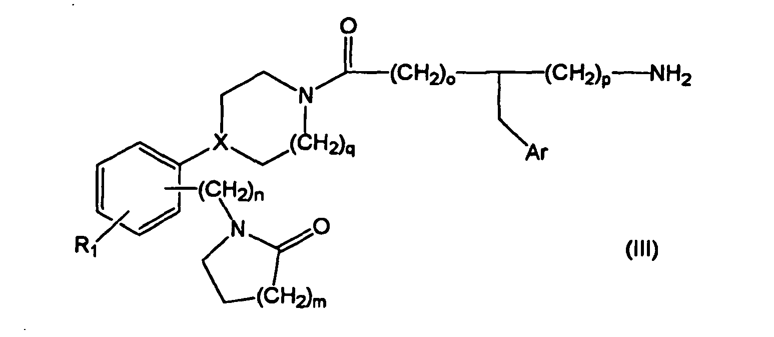
wherein R₁ is
   hydrogen,
   hydroxy,
   cyano,
   nitro,
   halo,
   straight chain or branched C₁-C₈ alkyl
   straight chain or branched C₁-C₈ alkoxy or
   straight chain or branched halo C₁-C₈ alkyl;
Ar is aryl or heteroaryl which may both be unsubstituted or substituted with one to three substituents independently selected from the group consisting of cyano, nitro, perfluoroalkoxy, halo, alkyl, (D)-cycloalkyl, alkoxy and haloalkyl wherein
aryl is an aromatic mono- or polycyclic moiety with 6 to 20 carbon atoms and heteroaryl is an aromatic moiety having 6 to 20 carbon atoms with at least one saturated, unsaturated or aromatic ring containing at least one heteroatom selected from O, N and/or S and 1 to 6 carbon atoms;
X is CH or N;
n is 1 - 4;
m is 0 - 3;
o is 0 - 2;
p is 0 - 2; and
q is 1 or 2.

*Compounds according to structural formula (I) are defined as follows*. 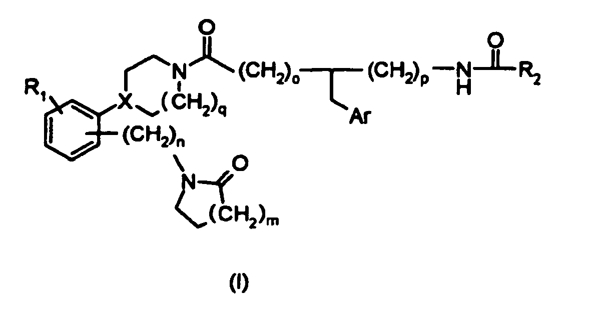 or a pharmaceutically acceptable salt or solvate thereof, wherein
Ar is:
   aryl or heteroaryl which may both be substituted or unsubstituted;
R₁ is independently:
   hydrogen,
   hydroxy,
   cyano,
   nitro,
   halo,
   alkyl,
   alkoxy or
   haloalkyl;
R₂ is: 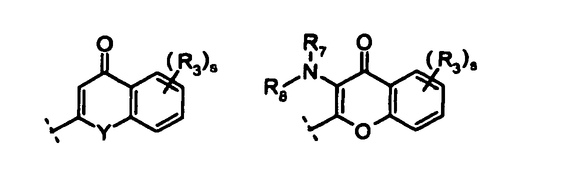
each R₃ is independently:
   hydrogen,
   halo,
   alkyl,
   haloalkyl,
   alkoxy,
   (D)-cycloalkyl,
   (D)-aryl,
   (D)-heteroaryl or
   (D)-heterocyclyl,
   wherein heterocyclyl excludes a heterocyclyl containing a single nitrogen,
   wherein aryl, heteroaryl, heterocyclyl, alkyl and/or cycloalkyl may be substituted or unsubstituted;
R₇ and R₈ are each independently:
   hydrogen,
   alkyl or
   cycloalkyl, or
   R₇ and R₈ together with the nitrogen to which they are attached form a 5- to 8-membered ring,
   wherein alkyl and cycloalkyl are both unsubstituted or substituted;
D is a bond or alkyl;
X is CH or N;
Y is O or NR₇;
n is 1 - 4;
m is 0 - 3;
o is 0 - 2;
p is 0 - 2;
q is 1 or 2;
s is 0-5.

*Compounds according to formula (II) are defined as follows.* 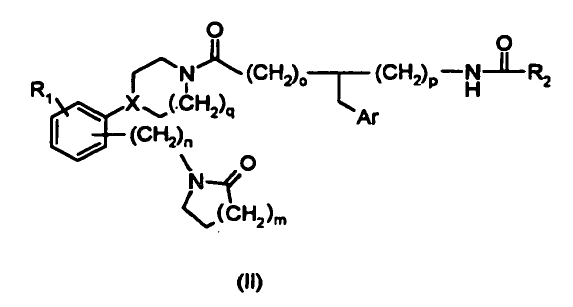 or a pharmaceutically acceptable salt or solvate thereof, wherein
Ar is:
   aryl or heteroaryl which may both be substituted or unsubstituted;
R₁ is independently:
   hydrogen,
   hydroxy,
   cyano,
   nitro,
   halo,
   alkyl,
   alkoxy or
   haloalkyl;
   R₂ is: 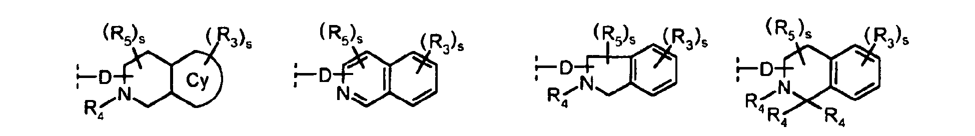 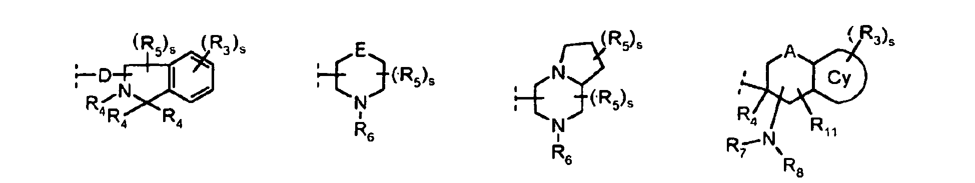 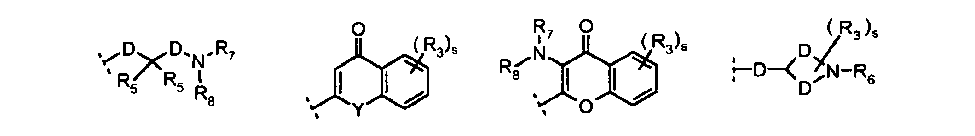
each R₃ is independently:
   hydrogen,
   halo,
   alkyl,
   haloalkyl,
   alkoxy,
   (D)-cycloalkyl,
   (D)-aryl,
   (D)-heteroaryl or
   (D)-heterocyclyl,
   wherein heterocyclyl excludes a heterocyclyl containing a single nitrogen,
   wherein aryl, heteroaryl, heterocyclyl, alkyl and/or cycloalkyl may be substituted or unsubstituted;
each R₄ is independently:
   hydrogen,
   alkyl,
   C(O)alkyl,
   SO₂alkyl,
   SO₂aryl,
   (D)-aryl or
   cycloalkyl;
each R₅ is independently:
   hydrogen,
   alkyl,
   (D)-aryl,
   (D)-heteroaryl,
   (CH₂)ₜN(R₇)₂,
   (CH₂)ₜNR₇C(O) alkyl,
   (CH₂)ₜNR₇SO₂alkyl,
   (CH₂)ₜSO₂N(R₇)₂,
   (CH₂)ₜ(O)ᵣC₁-C₈ alkyl,
   (CH₂)ₜ(O)ᵣ(CH₂)ₜNR₇COR₇,
   (CH₂)ₜ(O)ᵣ(CH₂)ₜNR₇SO₂R₇,
   (CH₂)ₜ(O)ᵣ-heterocyclyl or
   (CH₂)ₜ(O)ᵣ(alkylene)-heterocyclyl;
each R₆ is independently:
   hydrogen,
   alkyl,
   (D)-phenyl,
   C(O)alkyl,
   C(O)phenyl,
   SO₂-alkyl or
   SO₂-phenyl;
R₇ and R₈ are each independently:
   hydrogen,
   alkyl or
   cycloalkyl, or
   R₇ and R₈ together with the nitrogen to which they are attached form a 5- to 8-membered ring,
   wherein alkyl and cycloalkyl are both unsubstituted or substituted;
R₁₁ is:
   hydrogen or
   alkyl;
Cy is:
   aryl,
   heteroaryl,
   heterocyclyl or
   carbocyclyl;
A is a bond, O, S(O)ᵤ, NR₆ or CH₂;
D is a bond or alkyl;
E is O, S or NR₆;
X is CH or N;
Y is O or NR₇;
n is 1 - 4;
m is 0 - 3;
o is 0, 1 or 2, with the proviso that, if o is 0, then p is 1 or 2;
p is 0, 1 or 2, with the proviso that, if p is 0, then o is 1 or 2;
q is 1 or 2;
r is 0 or 1;
s is 0 - 5;
t is 0 - 8;
u is 0 - 2.

In the above, any of the preferred definitions for each variant can be combined with the preferred definition of the other variants.

In the above and the following, the employed terms have the meaning as described below:

Aryl is an aromatic mono- or polycyclic moiety with 6 to 20 carbon atoms which is preferably selected from phenyl, biphenyl, naphthyl, tetrahydronaphthyl, fluorenyl, indenyl and phenanthrenyl, more preferably from phenyl and naphthyl.

Heteroaryl is an aromatic moiety having 6 to 20 carbon atoms with at least one heterocycle and is preferably selected from thienyl, benzothienyl, naphthothienyl, furanyl, benzofuranyl, chromenyl, indolyl, isoindolyl, indazolyl, quinolyl, isoquinolyl, phthalazinyl, quinoxalinyl, cinnolinyl and quinazolinyl, more preferably from thienyl, furanyl, benzothienyl, benzofuranyl and indolyl.

Heterocyclyl is a saturated, unsaturated or aromatic ring containing at least one heteroatom selected from O, N and/or S and 1 to 6 carbon atoms and is preferably selected from thienyl, furyl, piperidinyl, pyranyl, pyrrolyl, imidazolyl, pyrazolyl, pyridyl, pyrazinyl, pyrimidinyl, pyridazinyl, isothiazolyl and isoxazyl, more preferably from pyridyl, piperidinyl, imidazolyl and pyrazinyl.

Carbocyclyl is a monocyclic or polycyclic ring system of 3 to 20 carbon atoms which may be saturated, unsaturated or aromatic.

Alkyl is straight chain or branched alkyl having preferably 1 to 8 carbon atoms, such as methyl, ethyl, n-propyl, isopropyl, n-butyl, isobutyl, tert-butyl, n-pentyl, isopentyl, neopentyl, hexyl or heptyl, more preferably 1 to 4 carbon atoms.

Cycloalkyl is an alkyl ring having preferably 3 to 8 carbon atoms, such as cyclopropyl, cyclobutyl, cyclopentyl, cyclohexyl or cycloheptyl, more preferably 3 to 6 carbon atoms.

Alkoxy is O-alkyl wherein alkyl is as defined above and has preferably 1 to 4 carbon atoms, more preferably 1 or 2 carbon atoms.

Halo or halogen is a halogen atom preferably selected from F, Cl, Br and I, more preferably from F, Cl and Br.

Haloalkyl is an alkyl moiety as defined above having preferably 1 to 4 carbon atoms, more preferably 1 or 2 carbon atoms, wherein at least one, preferably 1, 2 or 3 hydrogen atoms have been replaced by a halogen atom. Preferred examples are -CF₃ -CH₂CF₃ and -CF₂CF₃.

The compounds of structural formulas (I) and (II) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

### Optical Isomers - Diastereomers - Geometric Isomers - Tautomers

The compounds of structural formulas (I) and (II) contain one or more asymmetric centers and can occur as racemates and racemic mixtures, single enantiomers, diastereomeric mixtures and individual diastereomers. The present invention is meant to comprehend all such isomeric forms of the compounds of structural formulas (I) and (II).

Some of the compounds described herein may exist as tautomers such as keto-enol tautomers. The individual tautomers, as well as mixtures thereof, are encompassed within the compounds of structural formulas (I) and (II).

The compounds of structural formulas (I) and (II) may be separated into their individual diastereoisomers by, for example, fractional crystallization from a suitable solvent, for example, methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formulas (I) and (II) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic, manganous, potassium, sodium, zinc, salts and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, ptoluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formulas (I) and (II) are meant to also include the pharmaceutically acceptable salts.

### Utility

The compounds of formulas (I) and (II) are melanocortin receptor modulators and, as such, are useful in the treatment, contro or prevention of diseases, disorders or conditions responsive to the activation or inactivation of one or more of the melanocortin receptors including, but not limited to, MC-1R, MC-2R, MC-3R, MC-4R and MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance), hypertension, hyperlipidemia, osteoarthritis, cancer, gall bladder disease, sleep apnea, depression, anxiety, compulsion, neuroses, insomnia/sleep disorder, substance abuse, pain, male and female sexual dysfunction (including impotence, loss of libido and erectile dysfunction), fever, inflammation, immune-modulation, rheumatoid arthritis, skin tanning, acne and other skin disorders, neuroprotective and cognitive and memory enhancement, including the treatment of Alzheimer's disease.

Some compounds encompassed by formulas (I) and (II) show highly selective affinity for the melanocortin-4 receptor relative to MC-1R, MC-2R, MC-3R and MC-5R, which makes them especially useful in the prevention and treatment of cancer cachexia, muscle wasting, anorexia, anxiety, depression, obesity, as well as male and/or female sexual dysfunction, including erectile dysfunction. "Male sexual dysfunction" includes impotence, loss of libido and erectile dysfunction. "Female sexual dysfunction" can be seen as resulting from multiple components, including dysfunction in desire, sexual arousal, sexual receptivity and orgasm.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably the compounds of formulas (I) and (II) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia as well as other diseases or disorders for which the compounds of formulas (I) and (II) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, the compounds of the present invention are given in a dose range of 0.001 milligram to about 100 milligram per kilogram of body weight, preferably as a single dose orally, or as a nasal spray.

### Formulation

The compound of formulas (I) and (II) is preferably formulated into a dosage form prior to administration. Accordingly, the present invention also includes a pharmaceutical composition comprising a compound of formulas (I) and (II) and a suitable pharmaceutical carrier.

The present pharmaceutical compositions are prepared by known procedures using well-known and readily available ingredients. In making the compositions of the present invention, the active ingredient (a compound of formulas (I) and (II)) is usually mixed with a carrier or diluted by a carrier or enclosed within a carrier, which may be in the form of a capsule, sachet, paper or other container. When the carrier serves as a diluent, it may be a solid, semisolid, or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions or sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the compounds of formulas (I) and (II), the terms "A moiety", "B moiety", and "C moiety" are used below. This moiety concept is illustrated below:

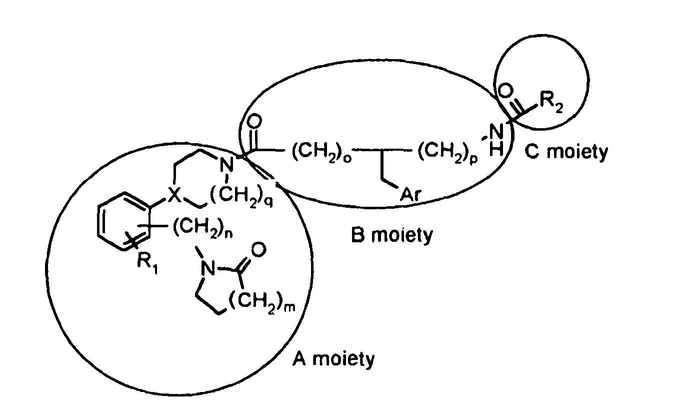

The preparation of the compounds of the present invention may be carried out via sequential or convergent synthetic routes. The skilled artisan will recognize that, in general, the three moieties of a compound of formulas (I) and (II) are connected via amide bonds. The skilled artisan can, therefore, readily envision numerous routes and methods of connecting the three moieties via standard peptide coupling reaction conditions.

The phrase "standard peptide coupling reaction conditions" means coupling a carboxylic acid with an amine using an acid activating agent such as EDC, dicyclohexylcarbodiimide or benzotriazol-1-yloxytris(dimethylamino)-phosphonium hexafluorophosphate, in an inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc or Fmoc are used extensively in the synthesis, and their removal conditions are well known to those skilled in the art. For example, removal of Z groups can be achieved by catalytic hydrogenation with hydrogen in the presence of a noble metal or its oxide, such as palladium on activated carbon in a protic solvent such as ethanol. In cases where catalytic hydrogenation is contraindicated by the presence of other potentially reactive functionality, removal of Z can also be achieved by treatment with a solution of hydrogen bromide in acetic acid or by treatment with a mixture of TFA and dimethylsulfide. Removal of Boc protecting groups is carried out in a solvent, such as methylene chloride, methanol or ethyl acetate, with a strong acid, such as TFA, HCl or hydrogen chloride gas.

The compounds of formulas (I) and (II), when existing as a diastereomeric mixture, may be separated into diastereomeric pairs of enantiomers by fractional crystallization from a suitable solvent such as methanol, ethyl acetate or a mixture thereof. The pair of enantiomers, thus obtained, may be separated into individual stereoisomers by conventional means using an optically active acid as a resolving agent. Alternatively, any enantiomer of a compound of formulas (I) and (II) may be obtained by stereospecific synthesis using optically pure starting materials or reagents of known configuration.

The compounds of formulas (I) and (II) of the present invention can be prepared according to the procedures of the following schemes and examples using appropriate materials and are further exemplified by the following specific examples. Moreover, by utilizing the procedures described herein, in conjunction with ordinary skills in the art, additional compounds of the present invention can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide or potassium hydroxide, and extraction of the liberated amine free base into an organic solvent, followed by evaporation. The amine free base, isolated in this manner, can be further converted into another pharmaceutically acceptable salt by dissolution in an organic solvent, followed by addition of the appropriate acid and subsequent evaporation, precipitation or crystallization. All temperatures are degrees Celsius. Mass spectra (MS) were measured by electron-spray ion-mass spectroscopy.

In the schemes, preparations and examples below, the various reagent symbols and abbreviations have the following meanings:
- BINAP: 2,2'-Bis(diphenylphosphino)-1,1'-binaphtyl
- Boc: t-butoxycarbonyl
- Bz₂O₂: dibenzoylperoxide
- DCM: dichloromethane
- DIPEA: diisopropylethylamine
- DMAP: 4-dimethylaminopyridine
- DME: dimethoxyethane
- DMF: N,N-dimethylformamide
- EDC: 1-(3-dimethylaminopropyl)-3-ethylcarbodiimide hydrochloride
- Et: ethyl
- EtOAc: ethyl acetate
- Fmoc: 9-fluorenylmethyl-carbamate
- HOAc: acetic acid
- HOAt: 1-hydroxy-7-azabenzotriazole
- HOBt: 1-hydroxybenzotriazole
- h: hour(s)
- NBS: N-bromosuccinimide
- NMM: N-methylmorpholine
- Me: methyl
- Ms: methanesulfonyl
- Pd₂(dba)₃: tris(dibenzylideneacetone) dipalladium(0)
- Phe: phenylalanine
- TFA: trifluoroacetic acid
- TEA: triethylamine
- Tic: 1,2,3,4-tetrahydroisoquinoline-3-carboxylic acid
- TMOF: trimethylorthoformate
- Z: benzyloxycarbonyl

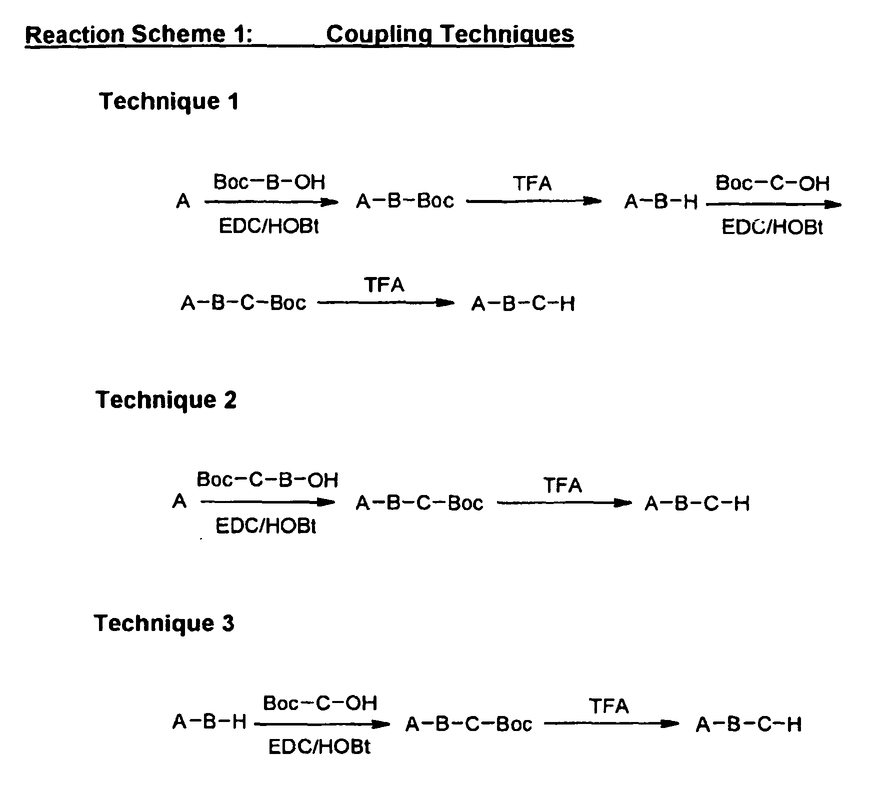

In coupling technique 1, an appropriate "A moiety" (e.g., 1-(2-piperazin-1-yl-benzyl)-pyrrolidin-2-one) is coupled to "B moiety" (e.g., L-Boc-p-Cl-Phe-OH) in the presence of EDC/HOBt followed by Boc deprotection. The coupled AB compound is then coupled to an appropriate "C moiety", followed by deprotection of Boc group and salt formation. Alternatively, when "C moiety" is not protected with Boc group, the final compound can be obtained without the deprotection step.

In coupling technique 2, an appropriate "AB moiety" is coupled to an appropriate "C moiety" in the presence of EDC/HOBt, followed by deprotection of Boc group and salt formation. Alternatively, when "C moiety" is not protected with Boc group, the final compound can be obtained without the deprotection step.

In coupling technique 3, an appropriate "BC moiety" is coupled to an appropriate "A moiety" in the presence of EDC/HOBt, followed by deprotection of Boc group and salt formation. Alternatively, when "C moiety" is not protected with Boc group, the final compound can be obtained without the deprotection step.

For coupling of A with Boc-B-OH, EDC/HOAt, EDC/HOBt or DCC/HOBt can be used.

Generally, the starting material of Boc-protected piperazine or piperidine (A moiety) can be deprotected in the presence of TFA/CH₂Cl₂, HCl/EtOAc, HCl/dioxane or HCl in MeOH/Et₂O, with or without a cation scavenger, such as dimethyl sulfide (DMS), before being subjected to the coupling procedure. It can be free-based before being subjected to the coupling procedure or, in some cases, used as the salt.

A suitable solvent such as CH₂Cl₂, DMF, THF or a mixture of the above solvents, can be used for the coupling procedure. A suitable base includes triethylamine (TEA), diisopropylethylamine (DIPEA), N-methylmorpholine (NMM), collidine and 2,6-lutidine. A base may not be needed when EDC/HOBt is used.

Generally after the reaction is completed, the reaction mixture can be diluted with an appropriate organic solvent, such as EtOAc, CH₂Cl₂ or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

Protecting groups such as Boc, Z, Fmoc or CF₃CO, can be deprotected in the presence of H₂/Pd-C, TFA/DCM, HCl/EtOAc, HCl/dioxane, HCl in MeOH/Et₂O, NH₃/MeOH or TBAF with or without a cation scavenger, such as thioanisole, ethane thiol or dimethyl sulfide (DMS). The deprotected amines can be used as the resulting salt or are free-based by dissolving in DCM and washing with aqueous bicarbonate or aqueous NaOH. The deprotected amines can also be free-based by ion exchange chromatography.

### Reaction Schemes for Preparation of "A moiety"

The "A moieties" of the present invention, in general, may be prepared from commercially available starting materials via known chemical transformations. The preparation of "A moiety" of the compound of the present invention is illustrated in the reaction scheme below. 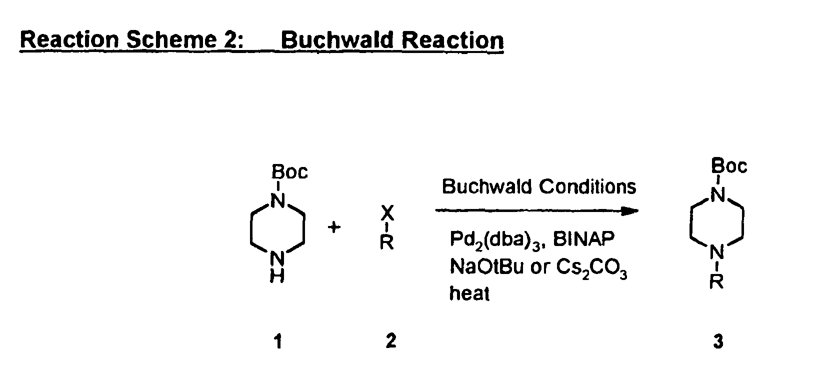 X = halo; and R is aryl

As shown in Reaction Scheme 2, the "A moiety" of the compounds of the present invention can be prepared by coupling halo-substituted aryl **2** (X-R) with 1-Boc-piperazine **1** in the presence of tri(dibenzylideneacetone) dipalladium (Pd₂(dba)₃), 2,2'-Bis(diphenylphosphino)-1,1'-binaphtyl (BINAP), and sodium-tert-butoxide (NaOtBu) or cesium carbonate (Cs₂CO₃) in an organic solvent, such as toluene, at a suitable temperature. More detailed examples of "A moiety" preparation are described below.

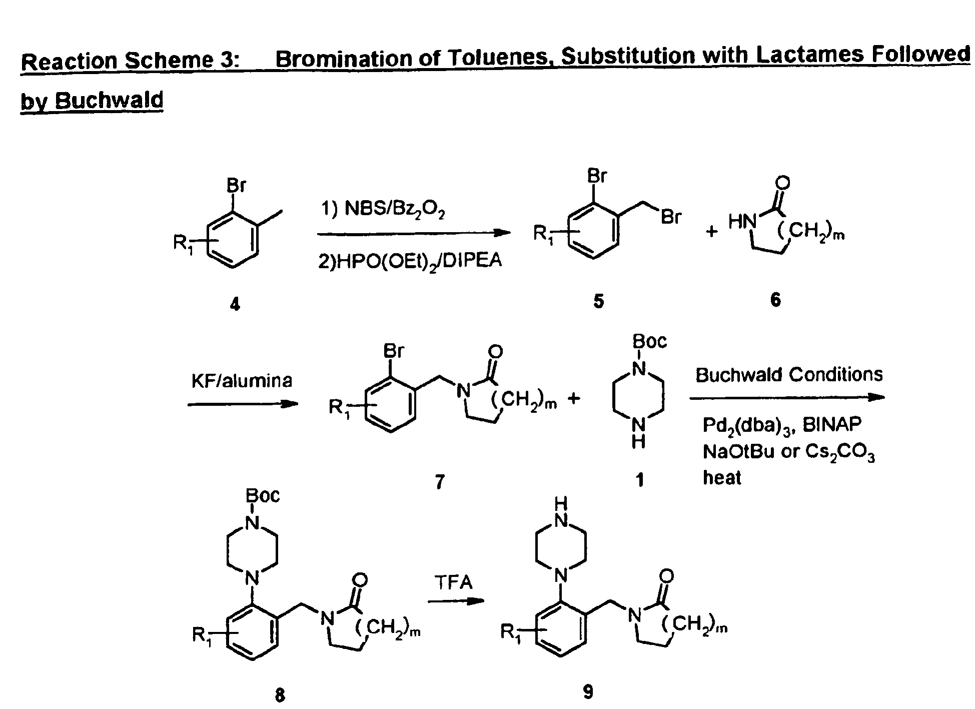

As shown in Reaction Scheme 3, the "A moiety" of the compounds of the present invention can be prepared by reacting various methyl benzenes **4** with NBS in the presence of a radical starter, such as Bz₂O₂, followed by reaction with diethyl phosphite in the presence of a base, such as DIPEA, to give benzylbromides **5,** which can then be used to alkylate lactames like **6,** in the presence of an appropriate base, such as KF/alumina. The substituted bromobenzenes can then be subjected to Buchwald conditions, followed by deprotection using an appropriate reactant, such as TFA. 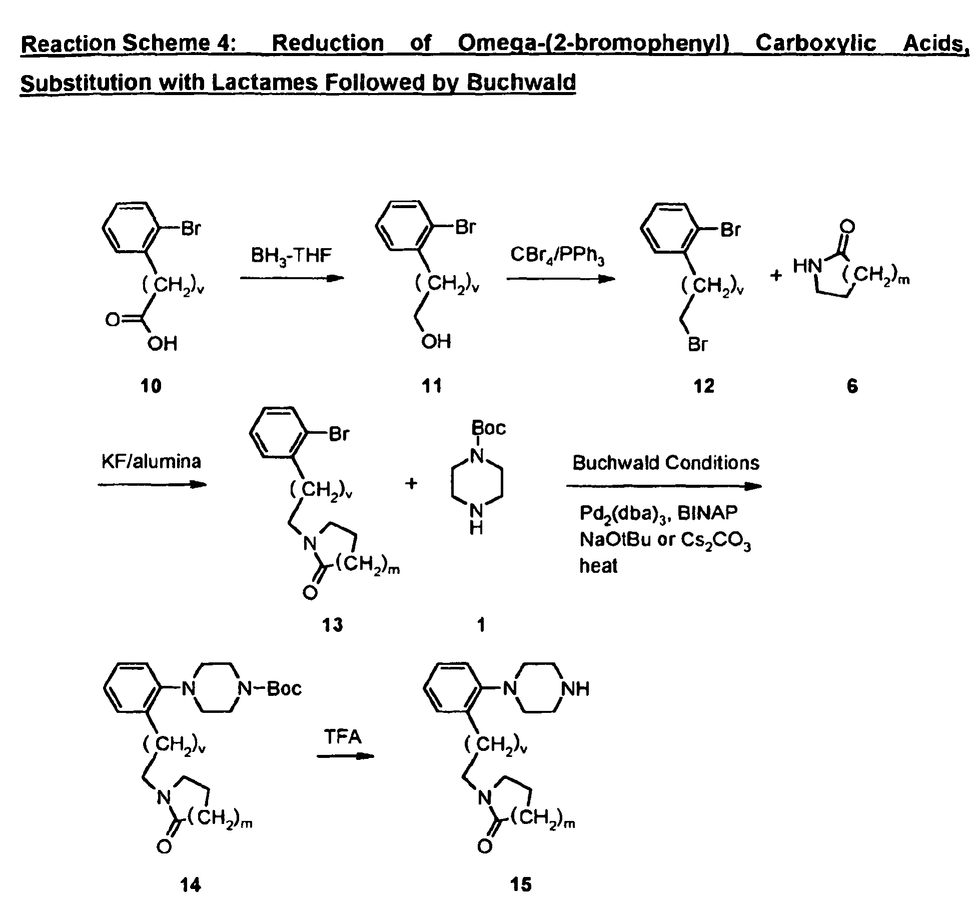 v = 0-2

As shown in Reaction Scheme 4, carboxylic acids **10** can be reduced to the corresponding alcohols **11** using an appropriate reagent such as BH₃-THF, which are subsequently transferred to the corresponding alkyl bromides **12** with reagents such as CBr₄ or PPh₃. The alkyl bromides can then be used to alkylate lactames like **6** in the presence of an appropriate base such as KF/alumina. The substituted bromobenzenes can then be subjected to Buchwald conditions followed by deprotection using an appropriate reactant such as TFA. 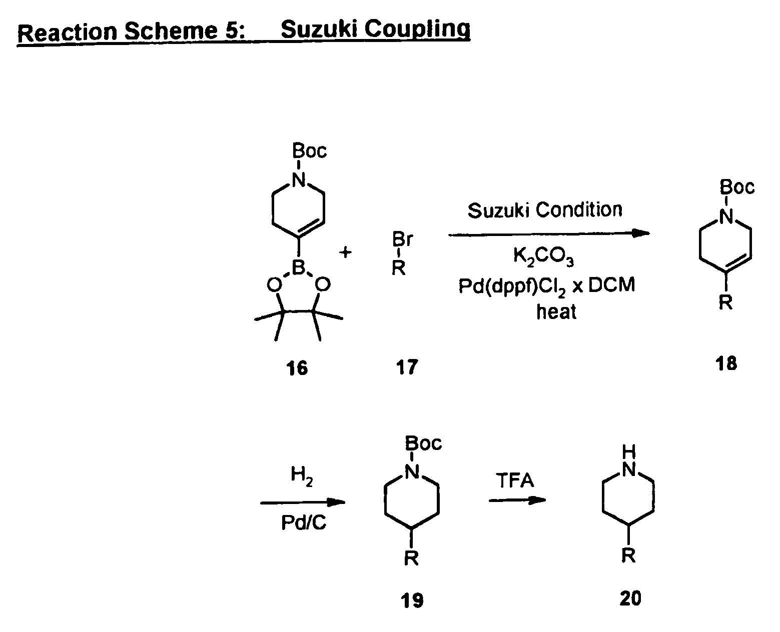 Br-R is compound 7 or 13

As shown in Reaction Scheme 5, 1-(2(H)-pyridine-carboxylic acid-3,6-dihydro-4-(4,4,5,5-tetramethyl-1,3,2-dioxaborolan-2-yl)-1,1-dimethyl ethyl ester **16** *(Tetrahedron Lett*. 2000, 41, 3705-3708) can be reacted with haloaromates such as **7** or **13** in the presence of a base such as K₂CO₃ and a catalyst such as dichloro(1,1'-bis(diphenylphosphino)-ferrocene)palladium(II) DCM adduct in an organic solvent such as DMF at a suitable temperature. The tetrahydropyridines can be hydrogenated in the presence of a catalyst such as Pd/C to yield the protected piperidines **19** which can subsequently be deprotected with a reagent such as TFA to yield piperidines **20.**

### Reaction Schemes for Preparation of "C moiety"

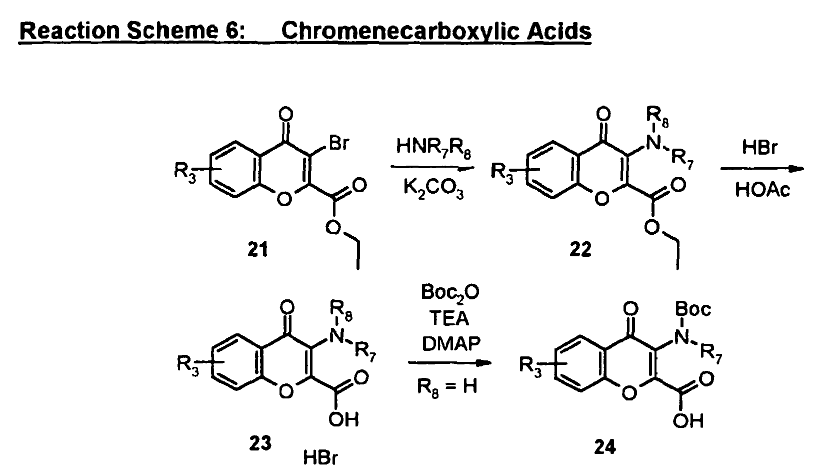

As shown in Reaction Scheme 6, ethyl 3-bromo-4-oxochromene-2-carboxylate **21** (*J. Chem. Soc. Perkin Trans.* / **1986**, 1643-1649) can be reacted with amines with or without a base such as K₂CO₃ in an appropriate solvent such as MeCN to form products **22** which are subsequently treated with a reagent such as HBr/HOAc to form carboxylic acids **23** When R₈ is hydrogen, the free amine can be protected with a reagent such as Boc₂O in the presence of TEA and DMAP in an appropriate solvent.

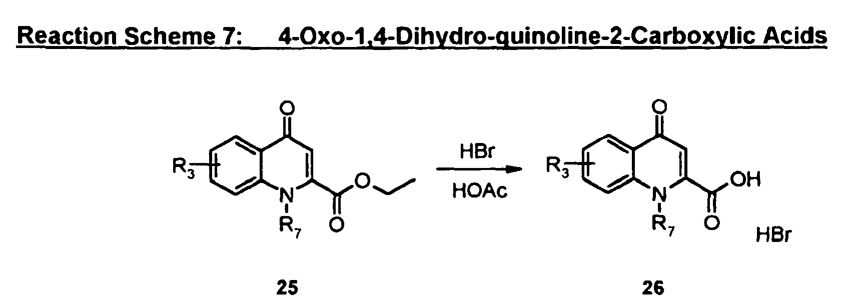

As shown in Reaction Scheme 7, ethyl 4-oxo-1,4-dihydro-quinoline-2-carboxylates **25** (*Bioorg. Med. Chem. Lett*. 2000, 10, 1487-1490) can be converted into the corresponding acids **26** by an appropriate reactant such as HBr/HOAc.

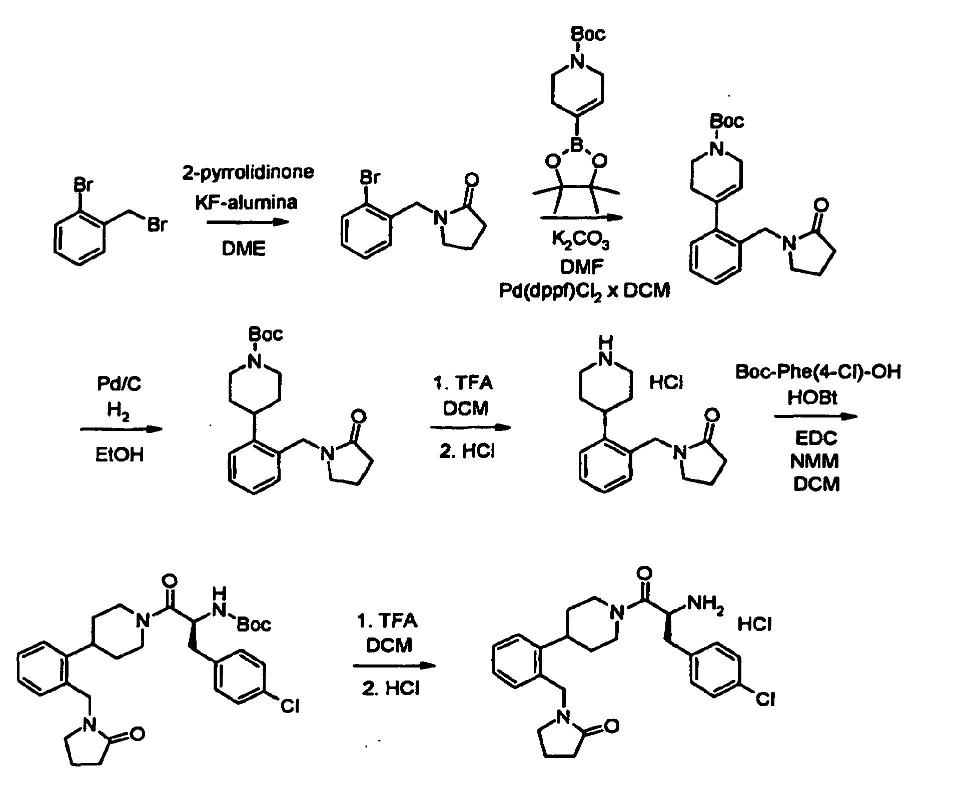

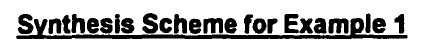

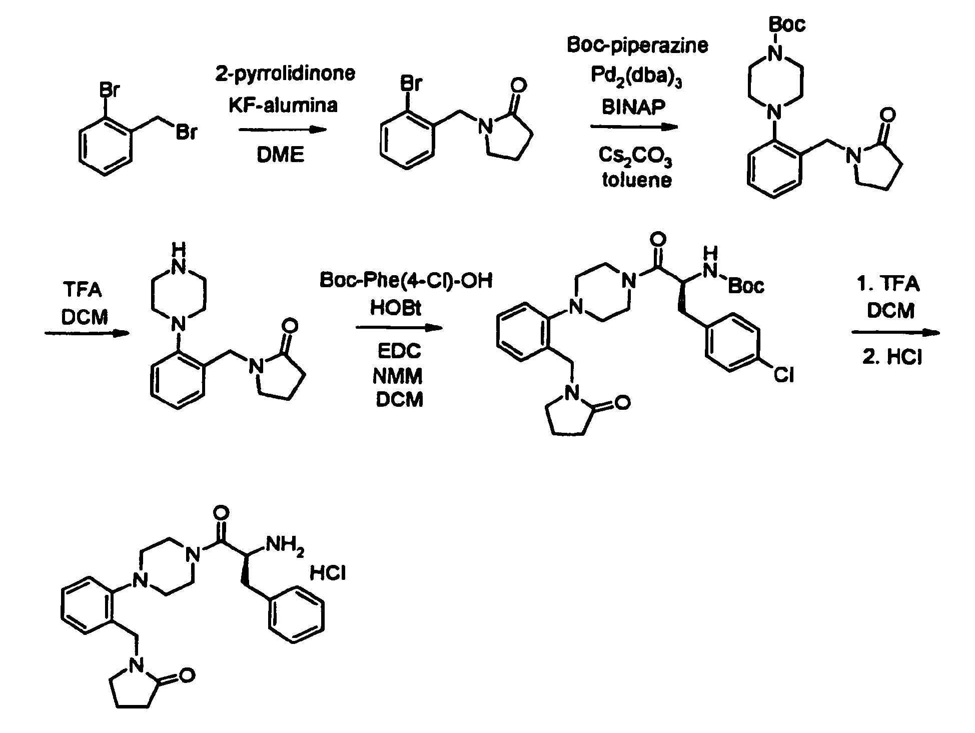

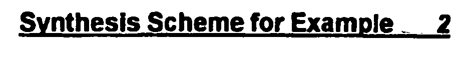

The following examples are provided to illustrate the invention and are not limiting the scope of the invention in any manner.

### Example 1:

### Intermediate 1a):

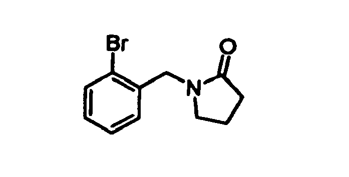

To a solution of 2-bromobenzyl bromide (3.05 g) and 2-pyrrolidinone (0.85 g) in DME (20 ml) was added KF-alumina (0.45 g) and the mixture was stirred for 48 h at room temperature. The inorganics were filtered off and the solvent was removed to afford the desired compound.

### Intermediate 1b):

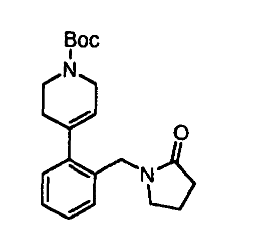

To intermediate 1a) (623 mg) in DMF (20 ml) was added 1-(2(H)-pyridine-carboxylic acid-3,6-dihydro-4-(4,4,5,5-tetramethyl-1,3,2-dioxaborolan-2-yl)-1,1-dimethyl ethyl ester (909 mg), dichloro(1,1'-bis(diphenylphosphino)-ferrocene)palladium(II) DCM adduct (108 mg) and K₂CO₃ (1002 mg). The reaction was heated to about 90°C overnight. The mixture was cooled, diluted with DCM and filtered through Celite. The filtrate was concentrated to dryness and the resulting residue was taken up in EtOAc (50 ml). The organics were washed with water, brine and concentrated to dryness. The crude product was purified by flash chromatography.

### Intermediate 1c):

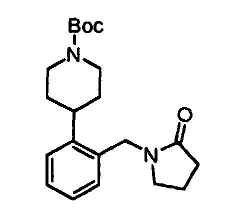

To intermediate 1b) (422 mg) in EtOH (20 ml) was added a slurry of 10% Pd/C in EtOH (20 ml). The mixture was stirred rapidly under H₂ (1 atm) for about 2 h. The reaction mixture was filtered over a pad of Celite and washed with EtOAc (100 ml). The filtrate was concentrated to dryness to yield the final compound.

### Intermediate 1d):

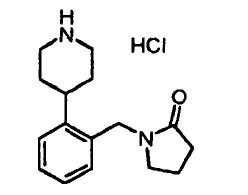

To the Boc-protected amine from 1c) (190 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml) was added. The precipitate was filtered and the residue was washed three times with ether and dried under reduced pressure to yield the desired compound.

### Intermediate 1e):

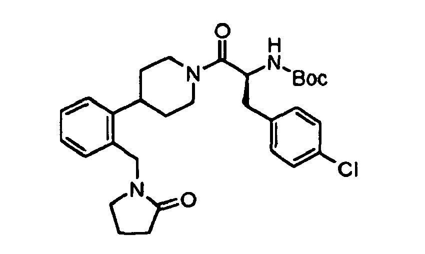

To Boc-L-4-chlorophenylalanine (82 mg) in DCM (5 ml) was added the amine hydrochloride from 1d) (42 mg), N-methylmorpholine (42 µl), HOBt (48 mg) and stirred for 20 min. EDC (72 mg) was added and stirring was continued for 1 h. An additional amount of N-methylmorpholine (20 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated in vacuo. Purification by column chromatography yielded the title compound.

### Compound 1:

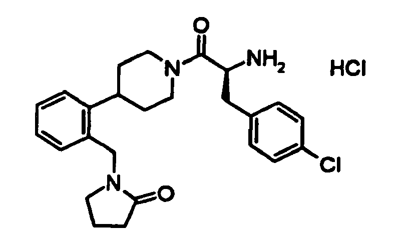

To the Boc-protected amine from 1e) (154 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml) was added. The precipitate was filtered and the residue was washed three times with ether and dried under reduced pressure to yield the desired compound.

### Example 2

### Intermediate 2a)

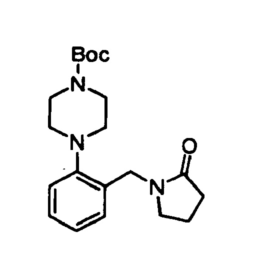

Boc-piperazine (895 mg), intermediate 1a) (1004 mg), Pd₂(dba)₃ (235 mg), BINAP (442 mg) and cesium carbonate (3 g) were mixed together in toluene (20 ml). The mixture was degassed and heated to 100°C for 3 d. The mixture was diluted with ether (100 ml) and filtered over Celite. The filtrate was concentrated and then subjected to chromatography on silica gel to yield the title compound.

### Intermediate 2b)

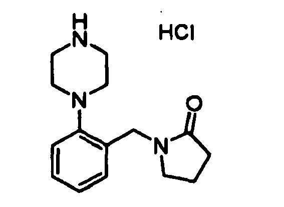

To the Boc-protected amine from 2a) (680 mg) in DCM (10 ml) was added TFA (2 ml) and stirred at room temperature for 90 min. Additional TFA (2 ml) was added and stirred for 10 min. The reaction mixture was diluted with DCM (20 ml) and carefully basified by pouring into 10% aqueous sodium carbonate solution (40 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (10 ml) and app. 1 M HCl in ether (20 ml) was added. The precipitate was filtered and the residue was washed three times with ether and dried under reduced pressure to yield the desired product.

### Intermediate 2c)

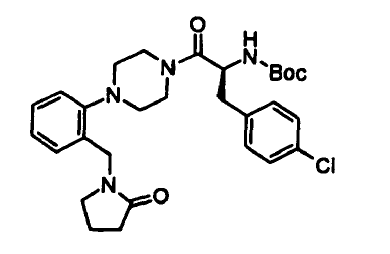

To Boc-L-4-chlorophenylalanine (82 mg) in DCM (5 ml) was added the amine hydrochloride from 2b) (61 mg), N-methylmorpholine (42 µl), HOBt (48 mg) and stirred for 20 min. EDC (72 mg) was added and stirring was continued for 1 h. An additional amount of N-methylmorpholine (20 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the title compound which was purified by column chromatography.

### Compound 2

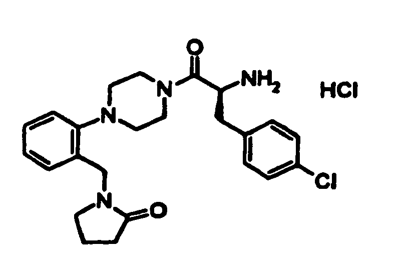

To the Boc-protected amino from 2c) (78 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml), was added. The precipitate was filtered and the residue was washed three times with ether and dried under reduced pressure to yield the desired product.

### Preparation of the carboxylic acids:

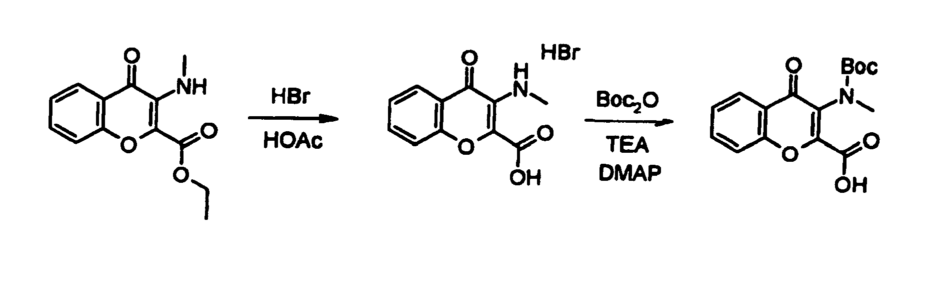

### Synthesis Scheme for Carboxylic Acid 1

### Carboxylic Acid 1:

To a solution of intermediate CA1a) (0.4 g) in THF (10 ml) was added TEA (670 µl), DMAP (20 mg), and Boc₂O (384 mg). The reaction mixture was stirred overnight. The solvent was removed under reduced pressure and the residue was dissolved in ethyl acetate. The solution was extracted with water, 1 M HCl and sat. NaHCO₃ and dried over Na₂SO₄. The solvent was removed under reduced pressure to yield the title compound.

### Intermediate CA1a):

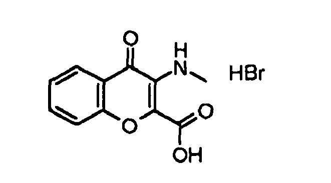

Ethyl 3-methylamino-4-oxochromene-2-carboxylate (0.8 g) was hydrolyzed by heating with hydrobromic acid (4 ml) and acetic acid (3 ml) for 4 h to give the desired compound after evaporation of the solvent.

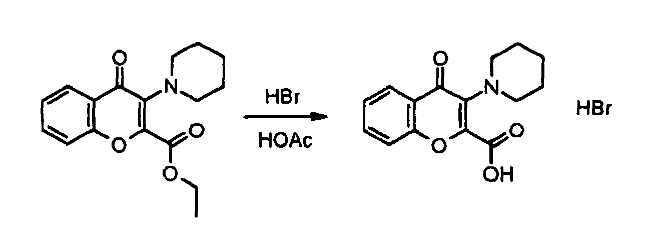

### Synthesis Scheme for Carboxylic Acid 2

### Carboxylic Acid 2:

Ethyl 3-piperidino-4-oxochromene-2-carboxylate (0.8 g) was hydrolyzed by heating with hydrobromic acid (4 ml) and acetic acid (3 ml) for 4 h to give the desired compound after evaporation of the solvent.

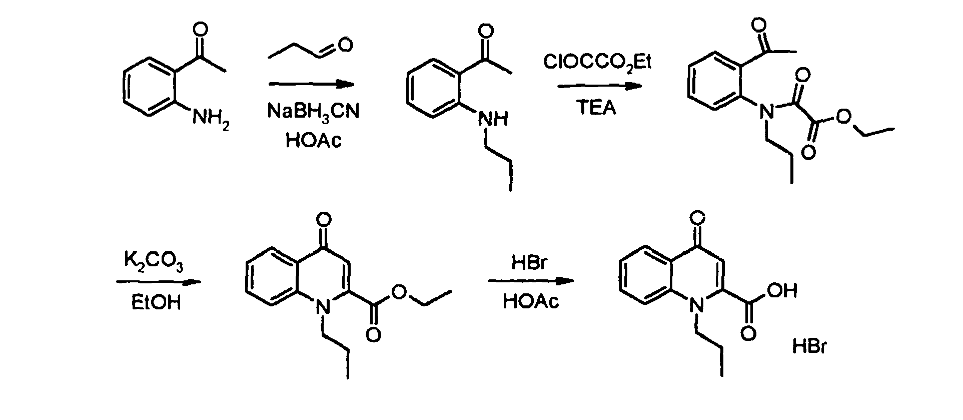

### Synthesis Scheme for Carboxylic Acid 3

### Carboxylic Acid 3:

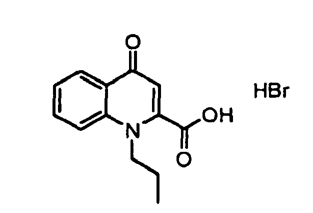

Intermediate CA3c) (0.1 g) was hydrolyzed by heating with hydrobromic acid (2 ml) and acetic acid (1.5 ml) for 3 h to give the desired compound after evaporation of the solvent.

### Intermediate CA3a):

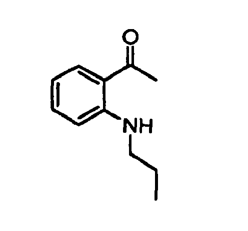

To a solution of 2'-aminoacetophenone (709 mg) in methanol (10 ml) was added propionaldehyde (580 µl) and TMOF (482 µl) and the solution was stirred overnight. The volatiles were removed under reduced pressure and the residue was redissolved in methanol (10 ml). Acetic acid (115 µl) and sodium cyanoborohydride (126 mg) were added and the reaction was stirred overnight. After basification with 1 M NaOH, the volatiles were removed under reduced pressure. The residue was dissolved in ethyl acetate and washed with water and brine. The organic phase was dried over Na₂SO₄ and the solvent was removed under reduced pressure to yield the title compound.

### Intermediate CA3b):

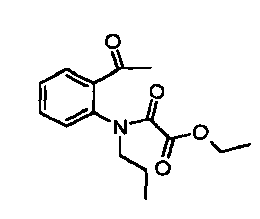

To a solution of intermediate CA3a) (681 mg) in dry THF (10 ml) was added TEA (670 µl) and the mixture was cooled to 0°C. At this temperature ethyl oxalyl chloride (470 µl) was added dropwise. The reaction mixture was stirred for 4 h at room temperature. The volatiles were removed under reduced pressure and the residue was dissolved in ethyl acetate. The solution was washed with water, sat. NaHCO₃ and brine and dried over Na₂SO₄ to yield the desired compound.

### Intermediate CA3c):

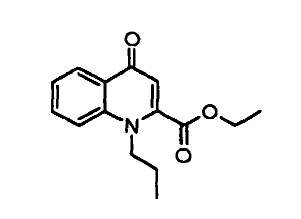

Intermediate CA3b) (555 mg) was dissolved in ethanol (10 ml). K₂CO₃ (276 mg) was added and the reaction mixture was stirred overnight. The reaction mixture was filtrated and the solvent was removed to yield the title compound.

### BIOLOGICAL ASSAYS

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 60 to 90 min at room temperature.

The degree of fluorescence polarization is determined with a fluorescence polarization microplate reader.

### B. Functional Assay

A functional cellular assay, based on competition between unlabeled cAMP and a fixed quantity of fluorescence labeled cAMP for a limited number of binding sites on a cAMP specific antibody, is used to discriminate melanocortin receptor agonists from antagonists by fluorescence polarization.

HEK293 cells expressing one of the human melanocortin receptors are grown in 384 well microtiter plates and are stimulated at different concentrations of the test compound to effect cAMP production. Cells are lysed and a fluorescence labeled cAMP conjugate is dispensed followed by the addition of anti-cAMP antibody used to detect the produced cAMP. The plate is read on a fluorescence polarization microplate reader and the amount of cAMP produced as a response to a test compound is compared to the production of cAMP resulting from stimulation with NDP-alpha-MSH.

The ability of a compound to block cAMP production, in response to NDP-alpha-MSH, is measured to define antagonistic activity of a test compound. Percent inhibition is determined by comparing the amount of cAMP produced in the presence to that produced in the absence of test compound.

### C. In Vivo Food Intake Models

### 1. Spontaneous Feeding Paradigm

Food intake in rats is measured after i.p. or p.o. administration of the test compound (see e.g. Chen, A.S. et al. Transgenic Res 2000 Apr 9(2):145-54)

### 2. Model of LPS- and Tumor-Induced Cachexia

Prevention or amelioration of cachexia induced by either lipopolysaccharide (LPS) administration or by tumor growth is determined upon i.p. or p.o. administration of test compounds to rats (see e.g. Marks, D.L.; Ling, N, and Cone, R.D. Cancer Res 2001 Feb 15;61(4):1432-8)

### D. Rat Ex Copula Assay

Sexually mature male Caesarian Derived Sprague Dawley (CD) rats (over 60 days old) are used with the suspensory ligament surgically removed to prevent retraction of the penis, back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body

Baseline and or vehicle evaluations are conducted to determine how and if an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired, 2 tailed t-tests, to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays, relevant to female sexual receptivity, include the behavioral model of lordosis and direct observations of copulatory activity. There is also a urethrogenital reflex model in anesthetized spinally transected rats for measuring orgasm in both male and female rats. These and other established animal models of female sexual dysfunction are described in McKenna KE et al, A Model For The Study of Sexual Function In Anesthetized Male And Female Rats, Am. J. Physiol. (Regulatory Integrative Comp. Physiol 30): R1276-R1285, 1991; McKenna KE et al, Modulation By Peripheral Serotonin of The Threshold For Sexual Reflexes In Female Rats, Pharm. Bioch. Behav., 40:151-156, 1991; and Takahashi LK et al, Dual Estradiol Action In The Diencephalon And The Regulation of Sociosexual Behavior In Female Golden Hamsters, Brain Res., 359:194-207, 1985.

Representative compounds of the present invention were tested and found to bind to the melanocortin-4 receptor. These compounds were generally found to have IC50 values less than 2 µM. Representative compounds of the present invention were also tested in the functional assay and found generally to activate the melanocortin-4 receptor with EC50 values less than 1 µM.

## Claims

1. The compounds of structural formula (III) 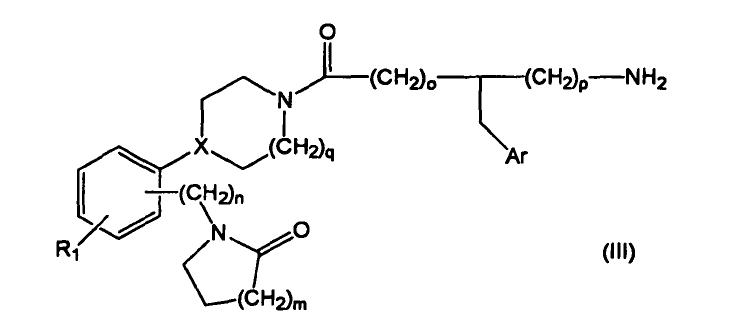
wherein R₁ is
hydrogen,
hydroxy,
cyano,
nitro,
halo,
straight chain or branched C₁-C₈ alkyl
straight chain or branched C₁-C₈ alkoxy or
straight chain or branched halo C₁-C₈ alkyl;
Ar is aryl or heteroaryl which may both be unsubstituted or substituted with one to three substituents independently selected from the group consisting of cyano, nitro, perfluoroalkoxy, halo, alkyl, (D)-cycloalkyl, alkoxy and haloalkyl, wherein
aryl is an aromatic mono- or polycyclic moiety with 6 to 20 carbon atoms and heteroaryl is an aromatic moiety having 6 to 20 carbon atoms with at least one saturated, unsaturated or aromatic ring containing at least one heteroatom selected from O, N and/or S and 1 to 6 carbon atoms;
X is CH or N;
n is 1 - 4;
m is 0 - 3;
o is 0 - 2;
p is 0 - 2; and
q is 1 or 2;
for use as a medicament.

2. Compounds of claim 1 for use in the treatment or prevention of disorders, diseases or conditions responsive to the inactivation or activation of the melanocortin-4 receptor in a mammal.

3. The compounds according to claim 2 for use in the treatment or prevention of cancer cachexia.

4. The compounds according to claim 2 for use in the treatment or prevention of muscle wasting.

5. The compounds according to claim 2 for use in the treatment or prevention of anorexia.

6. The compounds according to claim 2 for use in the treatment or prevention of anxiety and/or depression.

7. The compounds according to claim 2 for use in the treatment or prevention of obesity.

8. The compounds according to claim 2 for use in the treatment or prevention of diabetes mellitus.

9. The compounds according to claim 2 for use in the treatment or prevention of male or female sexual dysfunction.

10. The compounds according to claim 2 for use in the treatment or prevention of erectile dysfunction.

11. A pharmaceutical composition which comprises the compounds of claim 1 and a pharmaceutically acceptable carrier.

## Patentansprüche

1. Die Verbindungen der Strukturformel (III) 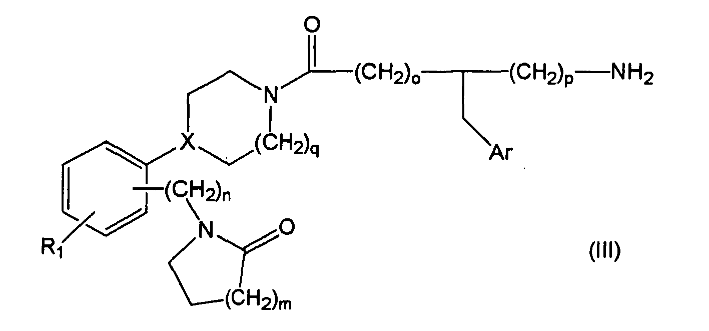
worin R₁
Wasserstoff,
Hydroxy,
Cyano,
Nitro,
Halogen,
geradkettiges oder verzweigtes C₁-C₈-Alkyl,
geradkettiges oder verzweigtes C₁-C₈-Alkoxy oder
geradkettiges oder verzweigtes Halogen-C₁-C₈-Alkyl ist;
Ar Aryl oder Heteroaryl ist, welche beide unsubstituiert oder mit ein bis drei Substituenten substituiert sein können, die unabhängig voneinander aus der Gruppe bestehend aus Cyano, Nitro, Perfluoralkoxy, Halogen, Alkyl, (D)-Cycloalkyl, Alkoxy und Halogenalkyl ausgewählt sind,
worin Aryl ein aromatischer mono- oder polycyclischer Baustein mit 6 bis 20 Kohlenstoffatomen ist und
Heteroaryl ein aromatischer Baustein mit 6 bis 20 Kohlenstoffatomen mit mindestens einem gesättigten, ungesättigten oder aromatischen Ring enthaltend wenigstens ein Heteroatom ausgewählt aus O, N und/oder S und 1 bis 6 Kohlenstoffatomen ist;
X CH oder N ist;
n 1 - 4 ist;
m 0 - 3 ist;
o 0 - 2 ist;
p 0 - 2 ist; und
q 1 oder 2 ist;
zur Verwendung als ein Arzneimittel.

2. Verbindungen des Anspruchs 1 für die Verwendung in der Behandlung oder Prävention von Funktionsstörungen, Krankheiten oder Bedingungen, die auf die Inaktivierung oder Aktivierung des Melanocortin-4-Rezeptors in einem Säugetier ansprechen.

3. Die Verbindungen gemäß Anspruch 2 für die Verwendung in der Behandlung oder Prävention von Krebskachexie.

4. Die Verbindungen gemäß Anspruch 2 für die Verwendung in der Behandlung oder Prävention von Muskelschwund.

5. Die Verbindungen gemäß Anspruch 2 für die Verwendung in der Behandlung oder Prävention von Anorexie.

6. Die Verbindungen gemäß Anspruch 2 für die Verwendung in der Behandlung oder Prävention von Angstzuständen und/oder Depressionen.

7. Die Verbindungen gemäß Anspruch 2 für die Verwendung in der Behandlung oder Prävention von Fettleibigkeit.

8. Die Verbindungen gemäß Anspruch 2 für die Verwendung in der Behandlung oder Prävention von Diabetes mellitus.

9. Die Verbindungen gemäß Anspruch 2 für die Verwendung in der Behandlung oder Prävention von gestörter männlicher oder weiblicher sexueller Dysfunktion.

10. Die Verbindungen gemäß Anspruch 2 für die Verwendung in der Behandlung oder Prävention von Erektionsstörungen.

11. Eine pharmazeutische Zusammensetzung, welche die Verbindungen von Anspruch 1 und einen pharmazeutisch akzeptablen Träger umfasst.

## Revendications

1. Composés de formule structurale (III) 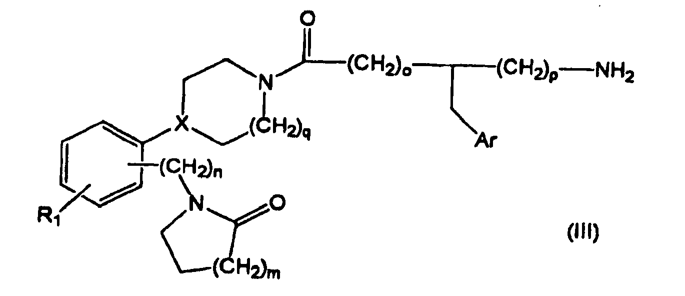 dans laquelle R₁ est
hydrogène,
hydroxy,
cyano,
nitro,
halogéno,
alkyle en C₁-C₈ à chaîne linéaire ou ramifié alcoxy en C₁-C₈ à chaîne linéaire ou ramifié ou halogénoalkyle en C₁-C₈ à chaîne linéaire ou ramifié ;
Ar est aryle ou hétéroaryle qui peuvent être tous les deux non substitués ou substitués par un à trois substituants choisis indépendamment dans le groupe constitué par cyano, nitro, perfluoroalcoxy, halogéno, alkyle, (D)-cycloalkyle, alcoxy et halogénoalkyle, où
aryle est un fragment aromatique mono- ou polycyclique ayant 6 à 20 atomes de carbone et hétéroaryle est un fragment aromatique ayant 6 à 20 atomes de carbone ayant au moins un cycle saturé, insaturé ou aromatique contenant au moins un hétéroatome choisi parmi 0, N et/ou S et 1 à 6 atomes de carbone ;
X est CH ou N ;
n est 1 - 4 ;
m est 0 - 3 ;
o est 0 - 2 ;
p est 0 - 2 ; et
q est 1 ou 2 ;
pour l'utilisation en tant que médicament.

2. Composés selon la revendication 1, pour l'utilisation dans le traitement ou la prévention de troubles, de maladies ou d'affections réagissant à l'inactivation ou à l'activation du récepteur de la mélanocortine-4 chez un mammifère.

3. Composés selon la revendication 2, pour l'utilisation dans le traitement ou la prévention d'une cachexie cancéreuse.

4. Composés selon la revendication 2, pour l'utilisation dans le traitement ou la prévention d'une fonte musculaire.

5. Composés selon la revendication 2, pour l'utilisation dans le traitement ou la prévention d'une anorexie.

6. Composés selon la revendication 2, pour l'utilisation dans le traitement ou la prévention d'une anxiété et/ou d'une dépression.

7. Composés selon la revendication 2, pour l'utilisation dans le traitement ou la prévention d'une obésité.

8. Composés selon la revendication 2, pour l'utilisation dans le traitement ou la prévention d'un diabète.

9. Composés selon la revendication 2, pour l'utilisation dans le traitement ou la prévention d'une dysfonction sexuelle masculine ou féminine.

10. Composés selon la revendication 2, pour l'utilisation dans le traitement ou la prévention d'une dysérection.

11. Composition pharmaceutique qui comprend les composés de la revendication 1 et un véhicule pharmaceutiquement acceptable.
